## Europäisches Patentamt

## European Patent Office

## Office européen des brevets

(19)

(11) Veröffentlichungsnummer: **0 216 874**
**B1**

(12)

# EUROPÄISCHE PATENTSCHRIFT

(45) Veröffentlichungstag der Patentschrift:
08.11.89

(51) Int. Cl.⁴: **C 12 P 7/06**

(21) Anmeldenummer: **86902271.5**

(22) Anmeldetag: **09.04.86**

(86) Internationale Anmeldenummer:
**PCT/AT 86/00031**

(87) Internationale Veröffentlichungsnummer:
**WO 86/06098 (23.10.86 Gazette 86/23)**

(54) **VERFAHREN ZUR GLEICHZEITIGEN PRODUKTION VON ALKOHOL UND PROTEINREICHEN FUTTERMITTELN.**

(30) Priorität: **09.04.85 AT 1069/85**

(43) Veröffentlichungstag der Anmeldung:
**08.04.87 Patentblatt 87/15**

(45) Bekanntmachung des Hinweises auf die Patenterteilung:
**08.11.89 Patentblatt 89/45**

(84) Benannte Vertragsstaaten:
**AT BE CH DE FR GB IT LI LU NL SE**

(56) Entgegenhaltungen:
**EP-A- 0 114 161**
**WO-A-81/03182**
**GB-A- 2 018 287**
**US-A- 2 663 154**

(73) Patentinhaber: **VOEST-ALPINE Aktiengesellschaft, Muldenstrasse 5, A-4020 Linz (AT)**

(72) Erfinder: **FALTEJSEK, Karl, Lüfteneggerstrasse 6, A-4020 Linz (AT)**
Erfinder: **CVITAS, Vilim, Wöberweg 8, A-4060 Leonding (AT)**
Erfinder: **HANKE, Reinhart, Annaberggasse 2, A-8700 Leoben (AT)**

(74) Vertreter: **Haffner, Thomas M., Dr. et al, Patentanwaltskanzlei Dipl.-Ing. Adolf Kretschmer Dr. Thomas M. Haffner Schottengasse 3a, A-1014 Wien (AT)**

## Beschreibung

Die Erfindung bezieht sich auf ein Verfahren zur gleichzeitigen Produktion von Alkohol und proteinreichen Futtermitteln, bei welchem stärkehaltige Feldfrüchte nach einer Zerkleinerung und Aufbereitung einer Verzuckerung und einer Hefegärung unterworfen werden. Als Aufbereitung kommt hier beispielsweise ein Laugen mit Dampf in Betracht. Für die Herstellung von Alkohol ist es bereits bekannt, stärkehaltige Feldfrüchte nach einer Zerkleinerung und Aufbereitung einer Verzuckerung zu unterwerfen. Die Gärung selbst wird üblicherweise durch Zusatz von Hefe vorgenommen und es sind Verfahren bekannt, bei welchen ein Hefeschlamm gebildet wird, welcher in weiterer Folge in Gärung gerät. Der gebildete Alkohol kann durch Destillation aus der Gärmaische abgetrennt werden. Die Gärmaische selbst wird unterschiedlichen Verwendungen zugeführt.

Stärkehaltige Feldfrüchte sind in der Regel als Vollnahrungsmittel deshalb wenig brauchbar, weil sie zumeist zu geringen Proteingehalt aufweisen.

Die Erfindung zielt nun darauf ab, ein Verfahren zur Herstellung von Alkohol aus stärkehaltigen Feldfrüchten dahingehend weiterzubilden, daß gleichzeitig aus den der Gärung nach der Zerkleinerung und Aufbereitung sowie Verzuckerung zuzuführenden stärkehaltigen Feldfrüchten ein proteinreiches Futtermittel hergestellt werden kann. Zur Lösung dieser Aufgabe besteht die Erfindung im wesentlichen darin, daß die Verzuckerung mit einer stärkereichen Teilmenge der zerkleinerten Feldfrüchte vorgenommen wird, worauf eine Suspension gebildet wird, daß die verzuckerte Suspension mit Hefemilch versetzt und einer Flotation unter Anwendung eines elektrischen Feldes unterworfen wird, worauf die mit Zucker beladene Hefe einer wenigstens zwei Stufen umfassenden Gärung unterworfen wird, wobei der Alkohol aus den Gärstufen destillativ abgetrennt wird und die Hefemilch aus der letzten Gärstufe einer Regenerierung unterworfen wird und zumindest teilweise der Flotation rückgeführt wird, und daß der die durch Flotation von der mit Zucker beladenen Hefe abgetrennte Resthefe enthaltende Schlamm sowie die Überschußhefe aus der Heferegenerierung mit der anderen Teilmenge der zerkleinerten Feldfrüchte versetzt und agglomeriert bzw. pelletiert wird. Dadurch, daß zunächst die einzusetzenden Feldfrüchte in Teilmengen unterteilt werden, wird die Voraussetzung geschaffen, daß innerhalb der gleichen Anlage zwei unterschiedliche Verfahrenswege, namentlich eine Gärung und eine Herstellung von Futtermitteln, ermöglicht wird. Die eine der beiden Teilmengen wird nun in konventioneller Weise nach der Verzuckerung und dem Versetzen mit Hefemilch einer Hefegärung unterworfen. Hiezu wird erfindungsgemäß vorgeschlagen, eine Flotation unter Anwendung eines elektrischen Feldes vorzunehmen, um auf diese Weise eine rasche Trennung der mit vergärbarem Zucker beladenen Hefe von dem unbeladenen Hefeschlamm zu erzielen. Dadurch, daß die mit Zucker beladene Hefe einer wenigstens zweistufig durchgeführten Gärung unterworfen wird, kann Alkohol in hoher Reinheit aus der zweiten Gärstufe destillativ abgetrennt werden und es wird der zusätzliche Vorteil erreicht, daß biologisches $CO_2$ in hoher Reinheit abgezogen werden kann. Die auf diese Weise sichergestellte hohe Reinheit der einzelnen Verfahrensprodukte erlaubt eine nahezu vollständige Kreislaufführung der Einsatzmaterialien. Alkohol wird aus der letzten Gärstufe erfindungsgemäß destillativ abgetrennt und die Hefemilch aus der letzten Gärstufe einer Regenerierung unterworfen. Auf Grund der hohen Reinheit und der auf Grund der zweistufigen Verfahrensführung in einfacher Weise einstellbaren Gärleistung und Reproduktionsrate der Hefebakterien kann ein mehr oder minder hoher Anteil Überschußhefe hergestellt werden, welcher zumindest teilweise der Flotation rückgeführt werden kann. Aus der Flotation kann in der erfindungsgemäßen Weise die von der mit Zucker beladenen Hefe abgetrennte Resthefe ebenso wie die Überschußhefe aus der Heferegenerierung mit einer weiteren Teilmenge der eingangs abgetrennten zerkleinerten Feldfrüchte versetzt werden, wodurch ein Gemisch entsteht, welches neben Stärke nunmehr einstellbare Anteile an Proteinen enthält. Die zerkleinerten Feldfrüchte können in der Folge gemeinsam mit der zugeführten Rest- bzw. Überschußhefe agglomeriert bzw. pelletiert werden, wodurch ein proteinreiches Futtermittel gewonnen wird.

In besonders bevorzugter Weise wird dieses Verfahren dadurch weitergebildet, daß die verzuckerte Stärkesuspension vor der Flotation zentrifugiert wird, worauf die abgeschiedenen Stärkereste und Ballaststoffe der zu agglomerierenden Fraktion zugemengt werden. Das Zentrifugieren vor der Flotationsstufe erlaubt die Abtrennung der nicht wasserlöslichen, nicht verzuckerten Anteile des Ausgangsproduktes, wodurch ein überaus reines Substrat für Beladung mit Hefe zur Verfügung gestellt wird. Mit Rücksicht auf die gleichzeitig vorgenommene Herstellung von proteinreichen Futtermitteln können die abgetrennten Stärkereste bzw. Ballaststoffe unmittelbar in die Futtermittelproduktion eingehen und auf diese Weise einer sinnvollen Verwendung zugeführt werden.

Es ist weiters vorteilhaft, aus der Flotationsstufe Resthefe abzuziehen, wobei auch hier wiederum in besonders einfacher Weise so vorgegangen werden kann, daß die Resthefe durch Zentrifugieren von der flüssigen wässerigen Phase abgetrennt und der zu agglomerierenden Fraktion zugemengt wird, und daß die flüssige Phase über wenigstens einen Wärmetauscher geleitet und der Verzuckerungsstufe rückgeführt wird. Durch diese Verfahrensführung wird eine bereits weitgehende Kreislaufführung sichergestellt. Die vollständige Ausnutzung der einzelnen Einsatzmaterialien kann noch dadurch verbessert werden, daß die der Verzuckerungsstufe rückgeführte flüssige Phase in zwei Teilmengen unterteilt wird, wobei die eine Teilmenge über einen Wärmetauscher aufgeheizt und die andere Teilmenge mit Enzymen für die Verzuckerung versetzt wird und beide Teilmengen der Verzuckerung rückgeführt werden. Eine derartige Verfahrensweise bietet erhebliche Vorteile im Hinblick auf den Zusatz der Enzyme für die Verzuckerung. Da die Verzuckerung bei relativ hohen Temperaturen vorgenommen werden muß, ist es erforderlich, zumindest eine Teilmenge der rückgeführten wässerigen Phase hoch zu erhitzen. Der Zusatz der Enzyme kann in eine Teilmenge erfolgen, welche nicht auf ähnlich hohe Temperaturen gebracht werden muß, wodurch sich Wärmeverluste bei der Aufgabe der Enzyme vermeiden lassen und sich der Zusatz der Enzyme unter günstigeren Arbeitsbedingungen vornehmen läßt.

Zur Gewinnung von biologisch reinem $CO_2$ kann in einfacher Weise aus der ersten Gärstufe über ein Sauggebläse $CO_2$ abgezogen und gegebenenfalls gemeinsam mit dem aus der destillativen Abtrennung von Alkohol aus der zweiten Gärstufe gewonnenen $CO_2$ einer weiteren Aufarbeitung, insbesondere einer Kompression und Abfüllung in Flaschen oder Kühlung, unterworfen werden.

Eine einfache Abtrennung der Überschußhefe, welche teilweise der Flotation und teilweise der Futtermittelproduktion zugeführt werden kann, kann dadurch erreicht werden, daß die Überschußhefe aus der Heferegenerierung zentrifugiert wird, daß die abgetrennten Feststoffe dem zu agglomerierenden Material zugesetzt werden und daß die flüssige Phase gemeinsam mit der aus der Flotation nach Zentrifugieren gewonnenen Phase rezirkuliert und der Verzuckerung rückgeführt wird.

Bei der destillativen Abtrennung des Alkohols fällt hoch gereinigtes Wasser an. Auch dieses hochreine Wasser kann im Rahmen des erfindungsgemäßen Verfahrens einer sinnvollen Verwendung zugeführt werden, wobei insbesondere vorgeschlagen wird, daß das bei der destillativen Abtrennung des Alkohols anfallende Wasser zum Teil der Verzuckerung rückgeführt und zum Teil mit Nährstoffen versetzt der Heferegenerierung rückgeführt wird. Hiebei kann das aus der Heferegenerierung gewonnene mit Nährstoffen, insbesondere Nährsalzen, angereicherte Wasser in besonders einfacher Weise als Pelletierwasser einer Pelletiereinrichtung zugeführt werden.

Zusammenfassend ergibt sich mit einem derartigen Verfahren eine vollständige Kreislaufführung, wobei aus dem Verfahrensablauf neben $CO_2$ und Alkohol proteinreiches Futtermittel abgezogen wird. In das Verfahren werden lediglich Enzyme vor der Verzuckerung und Nährstoffe vor der Heferegenerierung zugeführt, wobei alle weiteren Hilfsstoffe im Kreislauf geführt werden können.

Als stärkehaltige Feldfrüchte kommen beispielsweise Zuckerhirse, Korn, Getreide, Mais od. dgl. in Betracht.

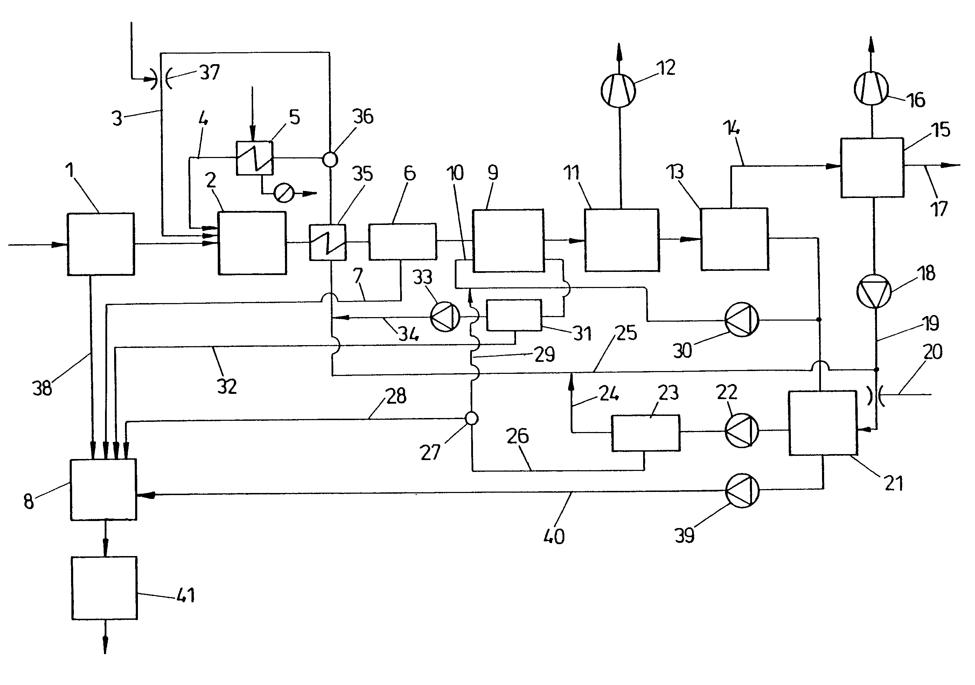

Die Erfindung wird nachfolgend an Hand einer in der Zeichnung schematisch dargestellten Anlage für die Durchführung des erfindungsgemäßen Verfahrens näher erläutert.

In der Zeichnung werden die stärkehaltigen Feldfrüchte einer Mahlung bzw. Aufbereitung bei 1 unterworfen. Das Stärkemehl gelangt in eine Verzuckerungsstufe 2, welcher über eine Leitung 3 enzymhaltiges Wasser und über eine Leitung 4 hocherhitzes Wasser bzw. Dampf zugeführt wird. Die Erhitzung des durch die Leitung 4 zugeführten Heißwassers erfolgt über einen mit Dampf betriebenen Wärmetauscher 5. Die aus der Verzuckerung abgezogene Suspension enthält noch nicht verzuckerte Anteile, welche in der Folge durch eine Zentrifuge 6 abgetrennt werden. Die abgetrennten Feststoffe, welche Stärkereste und Ballaststoffe darstellen, werden über eine Leitung 7 einer Pelletiereinrichtung 8 für die Herstellung proteinhältiger Futtermittel zugeführt. Die flüssige Phase aus der Zentrifuge 6 gelangt in der Folge in eine Flotationsstufe 9. Dieser Flotationsstufe 9 wird über eine Leitung 10 Hefeschlamm zugeführt, welcher in der Flotationsstufe mit Zucker beladen wird. Unter Anwendung eines elektrischen Feldes erfolgt eine rasche Trennung der mit Zucker beladenen Hefe von der verbleibenden unbeladenen Hefe, wobei die mit Zucker beladene Hefe in eine erste Gärstufe 11 übergeführt wird. Aus dieser ersten Gärstufe 11 kann mittels eines Sauggebläses 12 hochreines biologisches $CO_2$ abgezogen werden. Zur besseren Einstellbarkeit der Gärbedingungen wird die Gärung in mehrere Stufen unterteilt, wobei eine zweite Gärstufe mit 13 angedeutet ist. Sowohl in der Gärstufe 11 als auch in der Gärstufe 13 kann für eine gleichförmige Bewegung des Gärsubstrates durch geeignete Rührwerke Sorge getragen werden. Aus der Gärstufe 13 wird über eine Leitung 14 Äthanol, Wasserdampf und $CO_2$ abgetrennt, wobei aus der destillativen Trennung dieser Bestandteile, welche mit 15 schematisch angedeutet ist, über ein Sauggebläse 16 wiederum biologisch reines $CO_2$ abgezogen werden kann. Sowohl das Sauggebläse 12 als auch das Sauggebläse 16 erlaubt die Einstellung eines für die Gärung günstigen Druckes in den Gärstufen 11 und 13. Das durch die Destillation gewonnene Äthanol wird über die Leitung 17 abgezogen. Das verbleibende Wasser, welches ein hohes Maß an Reinheit aufweist, gelangt unter Zwischenschaltung einer Pumpe 18 über eine Leitung 19 zu einer Mischstelle, an welcher über Leitung 20 Nährstoffe zugesetzt werden. Die mit Nährstoffen versetzte wässerige Phase gelangt in eine Heferegenerierung 21, welcher die aus der zweiten bzw. letzten Gärstufe 13 abgezogene Hefe zugeführt wird. Der Hefeschlamm wird aus der Heferegenerierung 21 über eine Pumpe 22 abgezogen und in der Folge einer Zentrifuge 23 zugeführt. Durch Zentrifugieren wird hier eine wässerige Phase abgetrennt, welche über eine Leitung 24 einer Rezirkulationsleitung 25 rückgeführt wird. Die durch Zentrifugieren abgetrennte Trockenhefe wird über die Fördereinrichtung 26 einer Verteileinrichtung 27 zugeführt, wobei ein Teil der Trockenhefe über die Fördereinrichtung 28 als Überschußhefe der Pelletiereinrichtung 8 zugesetzt wird und ein anderer Teil der Trockenhefe über eine Fördereinrichtung 29 dem Hefeschlamm in der Leitung 10 zudosiert wird. Ein Teil der aus der Gärung abgezogenen Hefe wird hiebei unmittelbar im Kreislauf geführt, wofür eine Pumpe 30 vorgesehen ist.

Aus der Flotationsstufe 9 wird neben der mit Zucker beladenen Hefe, welche der Gärstufe 11 zugeführt wird, unbeladene Hefe abgezogen und einer Zentrifuge 31 zugeführt. Die auf diese Weise abgetrennte Resthefe wird über eine Fördereinrichtung 32 wiederum der Pelletiereinrichtung 8 zugeführt. Die wässerige Phase aus der Zentrifuge 31 wird über eine Pumpe 33 und eine Leitung 34 in die Rezirkulationsleitung 25 eingespeist und gemeinsam mit dem rezirkulieren Wasser einem Wärmeaustauscher 35 zugeführt. Die rezirkulierte Wassermenge wird bei 36 in zwei Anteile unterteilt, wobei der eine Anteil über den Wärmetauscher 5 hoch erhitzt wird und über die Leitung 4 der Verzuckerung rückgeführt wird, wohingegen der zweite Teil der rezirkulierten Wassermenge bei 37 mit Enzymen versetzt wird und über die Leitung 3 der Verzuckerung rückgeführt wird.

Aus der Mahlstufe und Aufbereitung 1 wurde, wie eingangs erwähnt, neben einer Teilmenge des Stärkemehles, welche der Verzuckerung zugeführt wurde, eine weitere Teilmenge abgetrennt, welche über eine Fördereinrichtung 38 der Pelletiereinrichtung zugeführt wird. Zusätzlich zu diesen stärkehältigen gemahlenen Ausgangsprodukten wird über die Fördereinrichtungen 28 und 32 Resthefe bzw. Überschußhefe zugesetzt. Über die Leitung 7, welche gleichfalls eine Fördereinrichtung sein kann, werden die aus der Zentrifuge 6 abgetrennten Stärkereste und Ballaststoffe der Pelletierstufe zugeführt. Den im wesentlichen trockenen Ausgangsprodukten wird nun noch Pelletierwasser zugesetzt. Zu diesem Zweck wird das aus der Heferegenerierung 21 abgezogene Wasser über eine Pumpe 39 und eine Leitung 40 der Pelletiereinrichtung zur Verfügung gestellt, womit ein mit Nährsalz angereichertes Wasser zur Herstellung eines hochwertigen proteinhaltigen Futtermittels auf der Basis von Stärkemehl Verwendung findet. Nach dem Agglomerieren bzw. Pelletieren in der Pelletierstufe 8 kann das proteinreiche Futtermittel einer Lagerung bzw. Absackung 41 zugeführt werden.

**Patentansprüche**

1. Verfahren zur gleichzeitigen Produktion von Alkohol und proteinreichen Futtermitteln, bei welchem stärkehaltige Feldfrüchte nach einer Zerkleinerung und Aufbereitung (1) einer Verzuckerung (2) und einer Hefegärung (11, 13) unterworfen werden, dadurch gekennzeichnet, daß die Verzuckerung (2) mit einer stärkereichen Teilmenge der zerkleinerten Feldfrüchte vorgenommen wird, worauf eine Suspension gebildet wird, daß die verzuckerte Suspension mit Hefemilch versetzt und einer Flotation (9) unter Anwendung eines elektrischen Feldes unterworfen wird, worauf die mit Zucker beladene Hefe einer wenigstens zwei getrennte Stufen umfassenden Gärung (11, 13) unterworfen wird, wobei der Alkohol aus den Gärstufen (11, 13) destillativ abgetrennt wird und die Hefemilch aus der letzten Gärstufe (13) einer Regenerierung (21) unterworfen wird und zumindest teilweise der Flotation (9) rückgeführt wird, und daß der die durch Flotation (9) von der mit Zucker beladene Hefe abgetrennte Resthefe enthaltende Schlamm sowie die Überschußhefe aus der Heferegenerierung (21) mit der anderen Teilmenge der zerkleinerten Feldfrüchte versetzt und agglomeriert bzw. pelletiert wird.

2. Verfahren nach Anspruch 1, dadurch gekennzeichnet, daß die verzuckerte Suspension vor der Flotation (9) zentrifugiert wird, worauf die abgeschiedenen Stärkereste und Ballaststoffe der zu agglomerierenden Fraktion zugemengt werden.

3. Verfahren nach Anspruch 1 oder 2, dadurch gekennzeichnet, daß aus der Flotationsstufe (9) Resthefe abgezogen wird, welche durch Zentrifugieren (31) von der flüssigen wässerigen Phase abgetrennt und der zu agglomerierenden Fraktion zugemengt wird, und daß die flüssige Phase über wenigstens einen Wärmetauscher (35) geleitet und der Verzuckerungsstufe (2) rückgeführt wird.

4. Verfahren nach Anspruch 3, dadurch gekennzeichnet, daß die der Verzuckerungsstufe (2) rückgeführte flüssige Phase in zwei Teilmengen unterteilt wird, wobei die eine Teilmenge über einen Wärmetauscher (5) aufgeheizt und die andere Teilmenge mit Enzymen für die Verzuckerung versetzt wird und beide Teilmengen der Verzuckerung (2) rückgeführt werden.

5. Verfahren nach einem der Ansprüche 1 bis 4, dadurch gekennzeichnet, daß aus der ersten Gärstufe (11) über ein Sauggebläse (12) $CO_2$ abgezogen und gegebenenfalls gemeinsam mit dem aus der destillativen Abtrennung von Alkohol aus der zweiten Gärstufe (13) gewonnenen $CO_2$ einer weiteren Aufarbeitung, insbesondere einer Kompression und Abfüllung in Flaschen oder Kühlung, unterworfen wird.

6. Verfahren nach einem der Ansprüche 1 bis 5, dadurch gekennzeichnet, daß die Überschußhefe aus der Heferegenerierung (21) zentrifugiert wird, daß die abgetrennten Feststoffe dem zu agglomerierenden Material zugesetzt werden und daß die flüssige Phase gemeinsam mit der aus der Flotation (9) nach Zentrifugieren gewonnenen Phase rezirkuliert und der Verzuckerung (2) rückgeführt wird.

7. Verfahren nach einem der Ansprüche 1 bis 6, dadurch gekennzeichnet, daß das bei der destillativen Abtrennung des Alkohols anfallende Wasser zum Teil der Verzuckerung (2) rückgeführt und zum Teil mit Nährstoffen versetzt und der Heferegenerierung (21) rückgeführt wird.

8. Verfahren nach einem der Ansprüche 1 bis 7, dadurch gekennzeichnet, daß das aus der Heferegenerierung (21) gewonnene mit Nährstoffen, insbesondere Nährsalzen, angereicherte Wasser als Pelletierwasser einer Pelletiereinrichtung (8) zugeführt wird.

**Claims**

1. Method for the simultaneous production of alcohol and high-protein foodstuffs, in which starch-containing cereals are subjected after crushing and handling (1) to saccharification (2) and yeast fermentation (11, 13), characterised in that the saccharification (2) is carried out with a starch-rich fraction of the crushed cereals, whereupon a suspension is formed, that the saccharified suspension is mixed with yeast milk and subjected to flotation (9) using an electric field, whereupon the yeast mixed with sugar is subjected to a fermentation comprising at least two separate stages (11, 13), the alcohol being separated by distillation from the fermentation stages (11, 13) and the yeast milk from the last fermentation stage (13) being subjected to regeneration (21) and returned at least in part to the flotation (9), and that the slurry containing the yeast residue separated by flotation (9) from the yeast mixed with sugar is, together with the surplus yeast from the yeast regeneration (21), added to the other fraction of the crushed cereals and agglomerated or pelletized.

2. Method according to Claim 1, characterised in that the saccharified suspension is centrifuged prior to the flotation (3), whereupon the separated starch residues and ballast matrials are mixed with the fraction to be agglomerated.

3. Method according to Claim 1 or 2, characterised in that yeast residue is drawn off from the flotation stage (9), separated by centrifuging (31) from the liquid aqueous phase and mixed with the fraction to be agglomerated, and that the liquid phase is passed through at least one heat exchanger (35) and returned to the saccharification stage (2).

4. Method according to Claim 3, characterised in that the liquid phase returned to the saccharification stage (2) is divided into two fractions, one of the fractions being heated up by a heat exchanger (5) and the other fraction mixed with enzymes for the saccharification and both fractions being returned to the saccharification (2).

5. Method according to any one of Claims 1 to 4, characterised in that $CO_2$ is drawn off from the first fermentation stage (11) via an exhauster (12) and, if appropriate, together with the $CO_2$ obtained from the second fermentation stage (13) from the separation by distillation of alcohol is subjected to further handling, in particular compression and decanting into bottles or cooling.

6. Method according to any one of Claims 1 to 5, characterised in that the surplus yeast from the yeast regeneration (21) is centrifuged, that the separated solids are added to the material to be agglomerated and that the liquid phase is recirculated together with the phase obtained from the flotation (9) after centrifuging and returned to the saccharification (2).

7. Method according to any one of Claims 1 to 6, characterised in that the water obtained during the separation by distillation of the alcohol is partly returned to the saccharification (2) and partly mixed with nutrients and returned to the yeast regeneration (21).

8. Method according to any one of Claims 1 to 7, characterised in that the water obtained from the yeast regeneration (21) and enriched with nutrients, in particular nutrient salts, is fed as pelletizing water to a pelletizing mill (8).

**Revendications**

1. Procédé de production simultanée d'alcool et de fourrages riches en protéines, selon lequel on soumet des produits amylacés des champs, après une fragmentation et une préparation (1), à une saccharification et à une fermentation par des levures (11, 13), procédé caractérisé en ce que la saccharification est réalisée sur une partie, riche en amidon, des produits des champs, fragmentés, ce qui provoque la formation d'une suspension; en ce qu'on ajoute du lait de levure à la suspension saccharifiée et on la soumet à une flottation (9) sous application d'un champ électrique, puis l'on soumet la levure chargée de sucre à une fermentation (11, 13) comportant au moins deux étapes séparées, l'alcool étant séparé par distillation des étapes de fermentation et le lait de levure provenant de la dernière étape de fermentation (13) étant soumis à une régénération (21) et étant recyclé au moins en partie vers la flottation et en ce que la suspension, contenant la levure résiduelle séparée par la flottation (9) de la levure chargée de sucre ainsi que la levure en excédent provenant de la régénération (21) de la levure, sont additionnées de l'autre partie des produits des champs, fragmentés, et sont agglomérées ou pastillées.

2. Procédé selon la revendication 1, caractérisé en ce que la suspension saccharifiée est centrifuguée avant la flottation (9) après quoi les restes d'amidon séparés et les substances d'accompagnement sont mélangés à la fraction à agglomérer.

3. Procédé selon la revendication 1 ou 2, caractérisé en ce qu'il est retiré de l'étape (9) de flottation de la levure résiduelle, que l'on sépare par centrifugation (91) de la phase aqueuse liquide et que l'on mélange à la fraction à agglomérer, et en ce qu'on fait passer la phase liquide par au moins un échangeur (35) de chaleur et l'on recycle cette phase vers l'étape de saccharification (2).

4. Procédé selon la revendication 3, caractérisé en ce qu'on subdivise en deux parties la phase liquide recyclée vers l'étape (2) de saccharification, on chauffe une partie à l'aide d'un échangeur (5) de chaleur et l'on ajoute à l'autre partie des enzymes pour la saccharification et l'on recycle les deux parties vers la saccharification (2).

5. Procédé selon l'une des revendications 1 à 4, caractérisé en ce que, de la première étape (11) de fermentation, du $CO_2$ est retiré à l'aide d'un ventilateur aspirant (12) et, éventuellement avec le $CO_2$ récupéré à partir de la séparation d'alcool, par distillation, de la seconde étape (13) de fermentation, il est soumis à un traitement supplémentaire de préparation, notamment à une compression et à une mise en bouteilles ou bien à un refroidissement.

6. Procédé selon l'une des revendications 1 à 5, caractérisé en ce que la levure excédentaire est centrifugée pour la séparer de la régénération de levure (21); en ce que les solides séparés sont ajoutés à la matière à agglomérer et en ce que la phase liquide est remise en circulation avec la phase récupérée, après centrifugation, de la flottation (9) et est recyclée vers la saccharification (2).

7. Procédé selon une des revendications 1 à 6, caractérisé en ce que l'eau, obtenue lors de la séparation de l'alcool par distillation, est recyclée en partie vers la saccharification (2) et est en partie additionnée de matières nutritives et est recyclée vers la régénération de la levure (21).

8. Procédé selon l'une des revendications 1 à 7, caractérisé en ce que l'eau, récupérée à partir de la régénération de levures (21) et enrichie en matières nutritives, notamment en sels nutritifs, est acheminée, à titre d'eau pour le pastillage, vers une installation de pastillage (8).